# EUROPEAN PATENT SPECIFICATION

(11) **EP 4 252 836 B1**
(45) Date of publication and mention of the grant of the patent: **02.09.2026**
(21) Application number: 21896744.6
(22) Date of filing: 03.11.2021
(51) Int. Cl.: A61N 5/10

(54) **NEUTRON CAPTURE THERAPY DEVICE AND CORRECTION METHOD THEREFOR**
NEUTRONENEINFANGTHERAPIEVORRICHTUNG UND KORREKTURVERFAHREN DAFÜR
DISPOSITIF DE THÉRAPIE PAR CAPTURE DE NEUTRONS ET SA MÉTHODE DE CORRECTION

(30) Priority: 25.11.2020 CN 202011334628
(43) Date of publication of application: 04.10.2023
(73) Proprietor: Neuboron Therapy System Ltd., Xiamen, Fujian 361026 (CN)
(72) Inventor: WANG, Chao, Nanjing, Jiangsu 211112 (CN); LIU, Yuan-hao, Nanjing, Jiangsu 211112 (CN)
(74) Representative: Gunzelmann, Rainer
(86) International application number: PCT/CN2021/128394
(87) International publication number: WO 2022/111243

(56) References cited:
- EP-A1- 2 805 745
- CN-A- 102 809 756
- CN-A- 104 174 121
- CN-A- 106 980 136
- CN-A- 110 361 766
- CN-A- 111 629 783
- JP-A- 2014 106 063

## Description

### TECHNICAL FIELD

The invention relates to the field of radioactive ray irradiation, and in particular to a neutron capture therapy device and a method for correcting the same.

### BACKGROUND

With the development of atomics, radioactive ray therapy, such as cobalt sixty, a linear accelerator, an electron beam, or the like, has become one of the major means to treat cancers. However, traditional photon or electron therapy is restricted by physical conditions of radioactive rays themselves, and thus will also harm a large number of normal tissues on a beam path while killing tumor cells. Furthermore, owing to different levels of sensitivity of tumor cells to radioactive rays, traditional radiotherapy often has poor treatment effect on malignant tumors (for example, glioblastoma multiforme and melanoma) with radio resistance.

In order to reduce radiation injury to normal tissues around tumors, a target therapy concept in chemotherapy is applied to radioactive ray therapy. With respect to tumor cells with high radio resistance, irradiation sources with high relative biological effectiveness (RBE), such as proton therapy, heavy particle therapy, neutron capture therapy, or the like, are also developed actively now. Here neutron capture therapy combines the abovementioned two concepts, for example, boron neutron capture therapy (BNCT), provides a better cancer treatment choice than traditional radioactive rays, by specific aggregation of boron-containing drugs in tumor cells in combination with precise neutron beam regulation and control.

During BNCT, an irradiation dose applied to a patient needs to be controlled accurately due to stronger neutron beam radioactive rays performing radiotherapy on the patient. Detection precision of a dose monitoring system directly affects a neutron irradiation dose applied to the patient actually.

A neutron dose detection method usually used in the dose monitoring system is a neutron activation method, but the neutron activation method needs to take a metal material out of an irradiation chamber after performing neutron beam irradiation on the metal material, and then measure a neutron irradiation dose. The method consumes time and may measure the neutron irradiation dose only after the neutron beam irradiation is interrupted, thus the neutron irradiation dose may not be obtained in real time. An active detector, such as a BF₃ counter tube, is used to detect neutron dose. Although the neutron dose may be detected in real time, contents of BF₃ in the counter tube is gradually reduced after BF₃ is irradiated by a certain dose of neutrons, so that sensitivity of the counter tube is reduced, and the neutron dose detected by the detector has errors, thus the neutron irradiation dose applied to the patient may not be accurately controlled.

The invention provides a neutron capture therapy device capable of measuring a neutron dose in real time and suppressing decrease of neutron beam measurement accuracy.

EP 2 805 745 A1 disclsoes a neutron capture therapy apparatus for irradiating a subject with neutron beams and a neutron beam measuring method. The signal processing circuit has a function of shaping the electrical signal from the gamma ray detector and outputting the result to the neutron dose measurement section and a noise removal function. The neutron beam measuring unit measures the amount of neutron beam irradiated to the scintillator in real time by counting the pulses of the pulse signal received from the signal processing circuit. The neutron dose measurement section receives the electrical signal shaped by the signal processing circuit and measures the dose of the neutron beam irradiated to a gold wire. The dose of the neutron beam measured by the neutron dose measurement section and the signal data of the scintillation detector measured by the neutron beam measuring unit are input to the correction coefficient calculation section. Then, the correction coefficient calculation section calculates the correction coefficient for matching the signal of the scintillation detector and the neutron dose with each other from the signal data of the scintillation detector and the dose of the neutron beam.

### SUMMARY

The invention is defined by the claims.

In order to solve the above problems, an aspect of the invention provides a neutron capture therapy device capable of applying an accurate neutron irradiation dose to a patient, and including a neutron dose detection device and a correction system configured to correct the neutron dose detection device. The neutron dose detection device includes a detector configured to receive neutrons and output electrical signals, a signal processing unit configured to process the electrical signals output from the detector and convert the electrical signals into pulse signals, and a counter configured to count the pulse signals output from the signal processing unit to obtain a counting rate.

Further, the correction system may periodically correct the neutron dose detection device.

Further, the correction system includes a metal part, a γ ray detection part configured to detect γ rays emitted by the metal part, and a correction coefficient calculation part, and the neutron capture therapy device corrects the neutron dose detection device based on a reaction rate of the metal part and the counting rate of the counter.

Further, the correction coefficient calculation part may calculate a correction coefficient k by formulas (2-1) and (2-2) as follows:
$k = \frac{T \times {RR}_{Au}}{{\sum }_{T} {C}_{t}} = \frac{{RR}_{Au}}{\overline{B}}$
${RR}_{Au} = \frac{λ \times C}{n \times ε \times Y \times {f}_{1} \times G \times \left(1-{e}^{λ {t}_{irr}}\right) \times {e}^{- {λt}_{c}} \times \left(1-{e}^{- {λt}_{m}}\right)}$
where *B̅* is an average counting rate recorded by the counter; T is time for a neutron beam to irradiate the detector and the metal part, with a unit of s; RR_{Au} is a reaction rate of the metal part; C is a peak gross count of γ rays measured by the γ ray detection part within a counting time; λ is a decay constant; n is the number of targets subject to irradiation; ε is a detection efficiency of the γ ray detection part for γ rays; Y is a γ ray branching ratio; f₁ is a self-absorption correction factor of γ rays; G is a flux fluctuation correction factor; tᵢᵣᵣ is irradiation time, with a unit of s; t_{c} is cooling time, with a unit of s; and tₘ is measurement time for γ energy spectrum, with a unit of s.

Further, the correction system may further include a correction part correcting a counting rate B in combination with the correction coefficient k, the corrected counting rate Bᵣ is calculated by using a formula (2-3) as follows:
${B}_{r} = B \times k$

Further, the neutron dose detection device may further include a conversion unit configured to convert a counting rate recorded by the counter into a neutron flux rate or a neutron dose rate, the conversion unit calculates a corrected neutron dose rate Dᵣ by using a formula (2-4) as follows:
${D}_{r} = \frac{{B}_{r}}{σ \times {f}_{2}} \times K \times N \times CBE$
where σ is a thermal neutron reaction cross-section (cm²); f₂ is a neutron attenuation correction factor induced by an activation detector; K is a boron dose conversion factor (Gy • cm²/ppm) for flux to 1 ppm boron concentration; N is an actual boron concentration (ppm); CBE is a composite biological effect factor.

Further, the neutron dose detection device may further include a neutron dose calculation unit configured to calculate the neutron flux rate or the neutron dose rate to obtain a neutron dose, the neutron dose calculation unit calculates a corrected neutron dose D_{acmr} by using a formula (2-5) as follows:
${D}_{{acm}_{r}} = \sum {D}_{r}$

Further, the metal part may be a ¹⁹⁷Au foil, and the γ ray detection part may be a high-purity germanium detector.

Further, the neutron capture therapy device may further include a neutron beam irradiation system configured to generate a neutron beam, and a control system configured to control the whole neutron beam irradiation process.

Further, the neutron beam irradiation system may include a neutron beam generation module, and a beam adjustment module configured to adjust the neutron beam generated by the neutron beam generation module and including a retarder, a reflector surrounding the retarder, and a radiation shield.

Another aspect of the invention provides a method performed before a treatment of a patient for correcting a neutron capture therapy device, including the following operations. Neutrons detected by a neutron dose detection device are received, and electrical signals are output. The electrical signals output from the neutron dose detection device are processed, and the electrical signals are converted into pulse signals. The pulse signals output from the signal processing unit are counted to obtain a counting rate. The neutron dose detection device is periodically corrected by using a correction system.

Further, the neutron dose detection device may be periodically corrected based on a reaction rate of a metal part and a counting rate of the neutron dose detection device.

In an embodiment, the method for correcting a neutron capture therapy device may further include the following operations. γ rays emitted by the metal part after neutron activation are detected. The reaction rate of the metal part is obtained by a measurement value of the γ rays.

A correction coefficient k is calculated based on the reaction rate of the metal part and the counting rate of the neutron dose detection device, and the counting rate of the neutron dose detection device is corrected by the correction coefficient k.

In other embodiments, the method for correcting a neutron capture therapy device may further include the following operations. A conversion unit converts the corrected counting rate of the neutron dose detection device into a neutron flux rate or a neutron dose rate.

### BRIEF DESCRIPTION OF THE DRAWINGS

FIG. 1 is a schematic diagram of a neutron beam irradiation system of a neutron capture therapy device of the invention.
FIG. 2 is a schematic diagram of a beam shaping body of a neutron capture therapy device of the invention.
FIG. 3 is a schematic diagram of a neutron beam irradiation system and a detection system of a neutron capture therapy device of the invention.
FIG. 4 is a schematic diagram of a neutron dose detection device and a correction system of a neutron capture therapy device of the invention.

### DETAILED DESCRIPTION

In order to make purposes, technical solutions and advantages of the invention more apparent and clearer, the invention will be further described in detail below in combination with the accompanying drawings and embodiments.

In the following descriptions, terms "first", "second", or the like, may be used here to describe various elements, but these elements are not limited by these terms, and these terms are only intended to distinguish the described objects without any sequential or technical meaning.

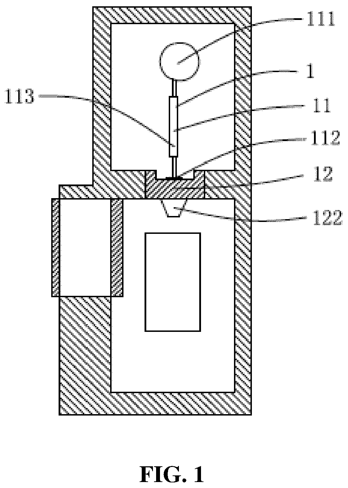

Radioactive ray therapy is a common means for treating cancers, and BNCT is an effective means for treating cancers and has been used increasingly in recent years. As shown in FIG. 1 to FIG. 4, a neutron capture therapy device irradiating a neutron beam of a preset neutron dose to a to-be-irradiated object, such as a patient S, so as to perform BNCT includes a neutron beam irradiation system 1, a detection system, a correction system 3 and a control system 4. The neutron beam irradiation system 1 is configured to generate a neutron beam suitable for performing the neutron irradiation therapy on the patient S. The detection system is configured to detect irradiation parameters such as a neutron dose, or the like, during the neutron beam irradiation therapy. The control system 4 is configured to control the whole neutron beam irradiation process.

BNCT produces two heavily charged particles ⁴He and ⁷Li by using a characteristic of a boron (¹⁰B)-containing drug having a high capture section for a thermal neutron, and through ¹⁰B(n,a)⁷Li neutron capture and a nuclear fission reaction. The two heavily charged particles each has an average energy of about 2.33 MeV, and has characteristics of high Linear Energy Transfer (LET) and a short range. The LET and range of the ⁴He particle are 150 keV/µm and 8 µm respectively, the LET and range of the ⁷Li heavily charged particle are 175 keV/µm and 5 µm respectively, and a total range of the two heavily charged particles is approximately equivalent to a size of a cell, so that radiation damage to an organism may be limited to the cell level. The boron-containing drug is selectively gathered in tumor cells. After the neutron beam enters a body of the patient S, it undergoes a nuclear reaction with boron in the body of the patient S, to produce two heavily charged particles ⁴He and ⁷Li, and the two heavily charged particles ⁴He and ⁷Li locally kill the tumor cells without causing too much damage to normal tissues.

As shown in FIG. 1, the neutron beam irradiation system 1 includes a neutron beam generation module 11 and a beam adjustment module 12 configured to adjust a neutron beam generated by the neutron beam generation module 11.

The neutron beam generation module 11 generates the neutron beam irradiated to the patient S, and includes an accelerator 111 configured to accelerate a charged particle beam, a target 112 configured to react with the charged particle beam to generate the neutron beam, and a charged particle beam transport part 113 located between the accelerator 111 and the target 112 and configured to transport the charged particle beam. The charged particle beam transport part 113 transports the charged particle beam to the target 112, and has one end connected to the accelerator 111 and the other end connected to the target 112. Furthermore, the charged particle beam transport part 113 is provided with a beam control device, such as a beam adjustment part (not shown), a charged particle scanning part (not shown), or the like. The beam adjustment part controls a travelling direction and a beam diameter of the charged particle beam. The charged particle beam scanning part scans the charged particle beam and controls an irradiation position of the charged particle beam relative to the target 112.

The accelerator 111 may be a cyclotron, a synchrotron, a synchrocyclotron, a linear accelerator, or the like. The commonly used target 112 includes lithium (Li) target and beryllium (Be) target. The charged particle beam is accelerated to an energy sufficient to overcome Coulomb repulsion of nuclei of the target 112 and undergoes a ⁷Li(p, n)⁷Be nuclear reaction with the target 112 to generate the neutron beam. The commonly discussed nuclear reaction includes ⁷Li(p, n)⁷Be and ⁹Be(p, n)⁹B. Usually, the target 112 includes a target layer and an anti-oxidation layer located on a side of the target layer and configured to prevent oxidation of the target layer, and the anti-oxidation layer is made of Al or stainless steel.

In the embodiments disclosed in the invention, the accelerator 111 accelerates charged particles to allow them to undergo a nuclear reaction with the target 112 to supply a neutron source. In other embodiments, the neutron source may be supplied by using a nuclear reactor, a D-T neutron generator, a D-D neutron generator, or the like. However, no matter whether the neutron source is supplied by accelerating the charged particles to allow them to undergo the nuclear reaction with the target 112, as disclosed in the invention, or the neutron source is supplied by the nuclear reactor, the D-T neutron generator, the D-D neutron generator, or the like, a mixed irradiation field is generated, that is, the generated beam includes a high-speed neutron beam, an epithermal neutron beam, a thermal neutron beam and a γ ray. During BNCT, the higher the content of the rest of irradiation rays (collectively referred to as irradiation ray contamination) except for the epithermal neutron, the greater the proportion of non-selective dose deposition in normal tissues, therefore radiation causing unnecessary dose deposition should be minimized.

The International Atomic Energy Agency (IAEA) has given five air beam quality factor recommendations for the neutron source used by clinical BNCT. The five recommendations may compare advantages and disadvantages of different neutron sources, and serve as a reference for selecting a neutron generation pathway and designing a beam shaping body 121. The five recommendations are as follows:
$Epithermal neutron flux > 1 \times {10}^{9} n / {cm}^{2} s$
$Fast neutron contamination < 2 \times {10}^{- 13} Gy - {cm}^{2} / n$
$Photon contamination < 2 \times {10}^{- 13} Gy - {cm}^{2} / n$
$Thermal to epithermal neutron flux ratio < 0.05$
$Epithermal neutron current to flux ratio > 0.7$

Note: an epithermal neutron has an energy region between 0.5 eV and 40 keV, a thermal neutron has an energy region less than 0.5 eV, and a fast neutron has an energy region greater than 40 keV.

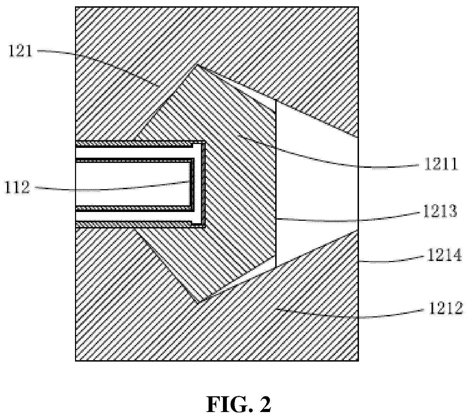

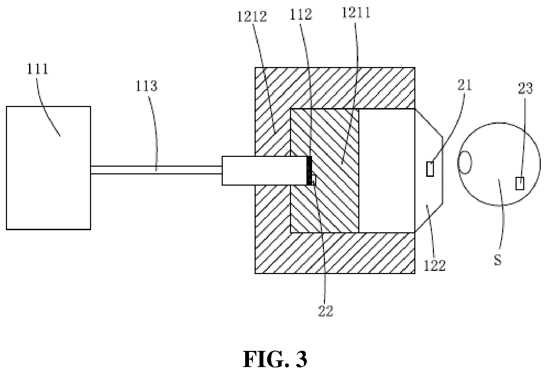

As shown in combination with FIG. 2 and FIG. 3, the beam adjustment module 12 is configured to adjust mixed irradiation rays generated in the neutron beam generation module 11, so as to minimize the irradiation ray contamination finally irradiated to the patient S and focus an epithermal neutron for treating the patient S to a part, required to be irradiated, of the patient S. The beam adjustment module 12 includes the beam shaping body 121 configured to decelerate and shield the neutron beam, and a collimator 122 configured to focus the epithermal neutron to the part, required to be irradiated, of the patient S. Specifically, the beam shaping body 121 includes a retarder 1211 configured to decelerate the neutron beam generated from the target 112 to an energy region of the epithermal neutron, a reflector 1212 surrounding the retarder 1211 and configured to guide a deviated neutron back to the retarder 1211 to increase a beam intensity of the epithermal neutron, a thermal neutron absorber 1213 configured to absorb a thermal neutron to avoid excessive dose deposition in superficial normal tissues during therapy, and a radiation shield 1214 configured to shield a leaked neutron and photon to reduce dose deposition in normal tissues at a non-irradiated region.

The retarder 1211 may be formed by stacking multiple different materials. The material of the retarder 1211 is selected according to factors, such as energy of the charged particle beam, or the like. For example, when energy of a proton beam from the accelerator 111 is 30 MeV and the Be target is used, the material of the retarder 1211 is lead (Pb), iron, aluminum (Al) or calcium fluoride. When the energy of the proton beam from the accelerator 111 is 11 MeV and the Be target is used, the material of the retarder 1211 is heavy water (D₂O), or lead fluoride, or the like. As a preferred embodiment, the retarder 1211 is formed by mixing MgF₂ and LiF which is 4.6% of MgF₂ by weight percentage, the reflector 1212 is made of Pb, and the thermal neutron absorber 1213 is made of ⁶Li. The radiation shield 1214 includes a photon shield and a neutron shield. Here the photon shield is made of Pb and the neutron shield is made of polyethylene (PE). The retarder 1211 may be formed in a biconical shape as disclosed in FIG. 2 or a cylindrical shape as disclosed in FIG. 3. The reflector 1212 is arranged around the retarder 1211, and has a shape adaptively changed according to the shape of the retarder 1211.

As shown in FIG. 3, the detection system includes a neutron dose detection device 21 configured to detect the neutron dose of the neutron beam in real time, a temperature detection device 22 configured to detect temperature of the target 112, a displacement detection device 23 configured to detect whether the patient S generates displacement during therapy, and a boron concentration detection device (not shown) configured to detect the boron concentration in the body of the patient S.

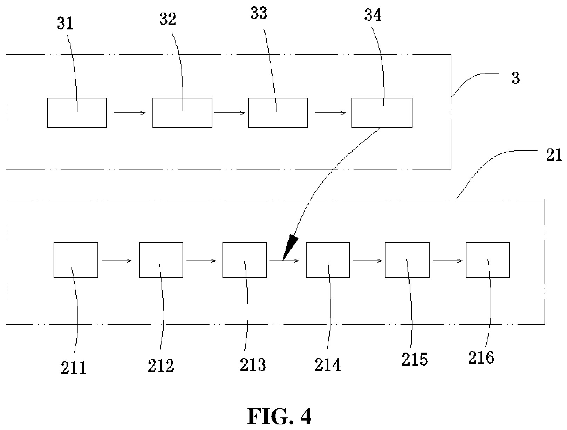

As shown in combination with FIG. 4, the neutron dose detection device 21 includes a detector 211 configured to receive neutrons and output electrical signals, a signal processing unit 212 configured to process the electrical signals output from the detector 211 and convert the electrical signals into pulse signals, a counter 213 configured to count the pulse signals output from the signal processing unit 212 to obtain a counting rate, a conversion unit 214 configured to convert a counting rate recorded by the counter 213 into a neutron flux rate or a neutron dose rate, a neutron dose calculation unit 215 configured to integrate the neutron flux rate or the neutron dose rate to obtain a neutron dose, and a display unit 216 configured to display the neutron dose.

In case that only the counting rate needs to be detected, the conversion unit 214, the neutron dose calculation unit 215, and the display unit 216 may not be provided, and the display unit 216 may not be configured to display related data in case of fully automatic control.

The detector 211 may be placed in the beam shaping body 121, may also be placed in the collimator 122, or may also be arranged at any position adjacent to the beam shaping body 121, as long as the position where the detector 211 is located may be configured to detect the neutron dose of the neutron beam.

The detector 211 capable of detecting the neutron dose of the neutron beam in real time is provided with an ionization chamber and a scintillation detector. Here a He-3 proportional counter, a BF₃ proportional counter, a fission chamber, and a boron ionization chamber use a structure of the ionization chamber as a substrate, and the scintillator detector contains an organic material or an inorganic material. When the thermal neutron is detected, the scintillator detector usually adds an element with a high capture section for a thermal neutron, such as Li, or B, or the like. A certain element in two types of detectors captures the neutron entering the detector or undergoes the nuclear fission reaction with the neutron entering the detector to release heavily charged particles and nuclear fission fragments, which generate a large number of ionization pairs in the ionization chamber or the scintillation detector, and these charges are collected and form electrical signals. The signal processing unit 212 performs noise reduction, conversion and separation processing on the electrical signals, and converts the electrical signals into pulse signals. A neutron pulse signal and a γ pulse signal are distinguished by analyzing a magnitude of a voltage pulse. The separated neutron pulse signal is continuously recorded by the counter 213 to obtain the counting rate (n/s) of the neutron. The conversion unit 214 calculates and converts the counting rate through internal software, programs, or the like, to obtain the neutron flux rate (cm⁻²s⁻¹), and further calculates and converts the neutron flux rate to obtain the neutron dose rate (Gy/s). Finally, the neutron dose calculation unit 215 integrates the neutron dose rate to obtain the real-time neutron dose.

A brief introduction is made below by example of the fission chamber, the scintillator detector and the BF₃ detector.

When the neutron beam passes through the fission chamber, it dissociates with gas molecules inside the fission chamber or a wall of the fission chamber to generate an electron and a positively charged ion, which are referred to as the ion pair as described above. Due to a high voltage of an electric field applied in the fission chamber, the electron moves towards a central anode wire and the positively charged ion moves towards a surrounding cathode wall, so that a measurable electrical signal is generated.

Substances, such as an optical fiber, or the like, in the scintillation detector absorb energy and then generate visible light, which uses ionizing radiation to excite an electron in a crystal or molecule to an excited state. Fluorescence emitted when the electron returns to a ground state is collected and then serves as detection of the neutron beam. The visible light emitted by action of the scintillation detector and the neutron beam is converted into an electrical signal by using a photomultiplier tube, to be output.

The BF₃ detector is placed in the beam shaping body 121 and configured to receive irradiation of the neutron beam, an element B in the BF₃ detector undergoes a nuclear reaction ¹⁰B(n, a)⁷Li with the neutron, and a particles generated by the nuclear reaction and ⁷Li electric particles are collected by a high voltage electrode under driving of the voltage, to generate electrical signals. The electrical signals are transmitted to the signal processing unit 212 through a coaxial cable, to be subject to signal amplification, filtering and shaping, so as to form pulse signals. The processed pulse signals are transmitted to the counter 213, to count pulses therein, so as to obtain the counting rate (n/s) through which intensity of the neutron beam, i.e., the neutron dose, may be measured in real time.

Here the conversion unit 214 calculates a neutron dose rate Dₜ by using a formula (1-1) as follows:
${D}_{t} = \frac{B}{σ \times {f}_{2}} \times K \times N \times CBE$
where B is a counting rate; f₂ is a neutron attenuation correction factor induced by an activation detector; σ is a thermal neutron reaction cross-section (cm²); K is a boron dose conversion factor (Gy • cm²/ppm) for flux to 1 ppm boron concentration; N is an actual boron concentration (ppm); CBE is a composite biological effect factor.

The neutron dose calculation unit 215 calculates a neutron dose Dacm irradiated to a to-be-irradiated object within a time t by using a formula (1-2) as follows:
${D}_{acm} = \sum {D}_{t}$

In a process of subject to the neutron beam irradiation, substances reacting with the neutron beam, such as an optical fiber, element B, or the like, within the detector 211, gradually reduce with increase of usage time, so that a proportion relationship between the electrical signal from the detector 211 and amount of the neutron beam actually irradiated to the detector 211 gradually changes, resulting in a deviation of the counting rate recorded by the counter 213, finally resulting in the neutron dose value detected from the neutron dose detection device 21 being less than the neutron dose actually irradiated to the detector 211. Therefore, the correction system 3 needs to be provided to periodically correct the neutron dose detection device 21. In general, the neutron dose detection device 21 is corrected by using the correction system 3 before each treatment, or in response to reaching a prescribed number of treatment, or after a prescribed period of time.

The correction system 3 includes a metal part 31, a γ ray detection part 32, a correction coefficient calculation part 33, and a correction part 34.

In the embodiments disclosed in the invention, the detector 211 is arranged at the head of the patient S, and the metal part 31 is arranged adjacent to the detector 211. In this condition, after the detector 211 and the metal part 31 are irradiated by the neutron beam for a certain period of time (for example, 10 minutes), the detector 211 and the metal part 31 are stopped from being irradiated by the neutron beam, the metal part 31 radiated after irradiation by the neutron beam is moved out of the irradiation chamber, the number of γ rays emitted by the metal part 31 is detected by the γ ray detection part 32, the correction coefficient calculation part 33 calculates a correction coefficient k by formulas (2-1) and (2-2) as follows:
$k = \frac{T \times {RR}_{Au}}{{\sum }_{T} {C}_{t}} = \frac{{RR}_{Au}}{\overline{B}}$
${RR}_{Au} = \frac{λ \times C}{n \times ε \times Y \times {f}_{1} \times G \times \left(1-{e}^{- λ {t}_{irr}}\right) \times {e}^{- λ {t}_{c}} \times \left(1-{e}^{- λ {t}_{m}}\right)}$
where *B̅* is an average counting rate recorded by the counter 213; T is time for a neutron beam to irradiate the detector 211 and the metal part 31, with a unit of s; RR_{Au} is a reaction rate of the metal part 31; λ is a decay constant; C is a peak gross count of γ rays measured by the γ ray detection part 32 within a counting time; ε is a detection efficiency of the γ ray detection part 32 for γ rays; Y is a γ ray branching ratio; f₁ is a self-absorption correction factor of γ rays; G is a flux fluctuation correction factor; tᵢᵣᵣ is irradiation time (sec), with a unit of s; t_{c} is cooling time, with a unit of s; tₘ is measurement time (sec) for γ energy spectrum, with a unit of s; and n is the number of targets subject to irradiation.

In the embodiments disclosed in the invention, the metal part 31 is a ¹⁹⁷Au foil, and the γ ray detection part 32 is a high-purity germanium detector. In other embodiments, a gold wire, a gold sheet, or the like may be used as the metal part 31, or a material which may be radiated by irradiation of the neutron beam, such as manganese or the like, may also be used, and a scintillator or the like may be used as the γ ray detection part 32.

In the embodiments disclosed in the invention, the correction system 3 corrects the counting rate recorded by the counter 213, correction may be achieved before not subjecting to conversion and calculation of the data, that is, without passing through the conversion unit 214 and the neutron dose calculation unit 215 firstly, thereby improving correction efficiency. That is, after the counting rate obtained by the counter, the counting rate is compared with the reaction rate after activation of the metal part to obtain the correction coefficient k. In other embodiments, the neutron dose rate or the neutron dose may be selected to be corrected according to a function relationship between the counting rate and the neutron dose rate or the neutron dose (see formulas (2-4) and (2-5) described below in detail). After the correction coefficient calculation part 33 calculates the correction coefficient k, the correction part 34 corrects a counting rate B in combination with the correction coefficient k, the corrected counting rate Bᵣ is calculated by using a formula (2-3) as follows:
${B}_{r} = B \times k$

After obtaining the corrected counting rate Bᵣ, the conversion unit 214 and the neutron dose calculation unit calculate a corrected neutron dose rate Dᵣ and a corrected neutron dose D_{acmr} by using formulas (2-4) and (2-5) respectively as follows:
${D}_{r} = \frac{{B}_{r}}{σ \times {f}_{2}} \times K \times N \times CBE$
${D}_{{acm}_{r}} = \sum {D}_{r}$

Since the neutron dose detection device 21 is periodically corrected by using the correction system 3, even if detection efficiency of the detector 211 is reduced, the counting rate recorded by the counter 213 of the detector 211 may be corrected by the correction system 3, thereby eliminating deviation of the measured neutron dose with respect to the actually irradiated neutron dose.

In the above embodiments, the time for performing correction may be set according to a specific situation, correction may be performed when treatment of the patient S is performed in the irradiation chamber, or correction may be performed when treatment of the patient S is not performed in the irradiation chamber.

The temperature detection device 22 is a thermocouple, and two conductors with different components (referred to as thermocouple wires or hot electrodes) are connected at both ends to form a loop. When temperature of the connection point is different, an electromotive force may be generated in the loop. This phenomenon is referred to as a thermoelectric effect, and the electromotive force is referred to as thermoelectric potential. The thermocouple performs temperature measurement by using this principle, of which one end directly configured to measure temperature of a medium is referred to as a working end (also known as a measurement end), and the other end is referred to as a cold end (also known as a compensation end). The cold end is connected to a display instrument or an assorted instrument, and the display instrument may indicate the thermoelectric potential generated by the thermocouple. Of course, as known by those skilled in the art, the temperature detection device 22 may also be any detector 211 capable of detecting temperature, such as a resistance thermometer, or the like.

The displacement detection device 23 is an infrared signal detector, and the infrared detector operates by detecting infrared rays emitted by a human body. The infrared detector collects infrared radiation from the outside and then gathers the infrared radiation on an infrared sensor. The infrared sensor usually uses a pyroelectric element, which releases charges to the outside when temperature of the infrared radiation changes, and an alarm is generated after detecting and processing charges. The detector 211 is aimed to detecting radiation of the human body. Therefore, a radiation-sensitive element must be very sensitive to infrared radiation with a wavelength of about 10 µm. Of course, it is well known by those skilled in the art that the displacement detection device 23 may be any detection device suitable for detecting change of displacement of a to-be-irradiated object, such as a displacement sensor. The displacement sensor determines whether the to-be-irradiated object moves, according to the change of displacement of the to-be-irradiated object relative to a certain reference object. It is also well known by those skilled in the art that the displacement detection device 23 not only may be configured to detect the change of displacement of the to-be-irradiated object, but also may be configured to detect change of displacement of a support member and/or a treatment table fixing the to-be-irradiated object, thereby indirectly knowing the change of displacement of the to-be-irradiated object.

During the neutron beam irradiation therapy for the patient S, boron is continuously supplied to the patient S as needed. A boron concentration may be detected by an inductively coupled plasma spectroscopy, a high-resolution a autoradiography, a charged ion spectroscopy, a neutron capture camera, a nuclear magnetic resonance imaging and a magnetic resonance imaging, a positive electron emission tomography, a prompt γ ray spectroscopy, or the like, and a device involved in the above detection method is referred to as a boron concentration detection device.

The invention is described by example of calculating the boron concentration in the body of the patient S by detecting γ rays released by the patient S. The neutron beam enters the body of the patient and reacts with boron to generate γ rays. By measuring the amount of γ rays, the amount of boron reacting with the neutron beam may be calculated, thereby calculating the boron concentration in the body of the patient S. The boron concentration detection device is configured to measure the boron concentration in the body of the patient S in real time when the neutron beam irradiation system 1 performs the neutron beam irradiation therapy on the patient S.

The boron concentration detection device detects γ rays (478 keV) generated by reaction between the neutron and boron, to measure the boron concentration, and a boron distribution measurement system (PG(Prompt-γ)-SPECT) capable of measuring a single-energy γ ray to measure distribution of the boron concentration is used as the boron concentration detection device. The boron concentration detection device includes a γ ray detection part 32 and a boron concentration calculation part. The γ ray detection part 32 is configured to detect information related to γ rays emitted from the body of the patient S, and the boron concentration calculation part calculates the boron concentration in the body of the patient S according to the information related to γ rays detected by the γ ray detection part 32. The γ ray detection part 32 may use the scintillator and various other γ ray detection devices. In the implementation, the γ ray detection part 32 is arranged in the vicinity of a tumor of the patient S, for example, at a position about 30 cm away from the tumor of the patient S.

The neutron capture therapy device disclosed in the invention is not limited to contents described in the above embodiments and structures shown in the drawings. The scope of protection of the invention is defined by the appended claims.

## Claims

1. A neutron capture therapy device comprising:
a neutron beam irradiation system (1) configured to generate a neutron beam, a neutron dose detection device (21) and a correction system (3) configured to correct the neutron dose detection device,
wherein the neutron dose detection device (21) comprises a detector (211) configured to receive neutrons of the neutron beam and output electrical signals, a signal processing unit (212) configured to process the electrical signals output from the detector and convert the electrical signals into pulse signals, and a counter (213) configurec to count the pulse signals output from the signal processing unit to obtain a counting rate; wherein the correction system (3) comprises a metal part (31) configured to be activated by neutrons of the neutron beam,
a γ ray detection par (32) configured to detect γ rays emitted by the metal part (31) activated by neutrons of the neutron beam to obtain a reaction rate of the metal part by a measurement value of the γ rays,
and a correction coefficient calculation part (33) configured to calculate a correction coefficient based on the reaction rate of the metal part and the counting rate obtained by the counter,
and wherein the correction system (3) is configured to correct by the correction coefficient the counting rate obtained by the counter.

2. The neutron capture therapy device of claim 1, wherein the correction system is configured to periodically correct the neutron dose detection device.

3. The neutron capture therapy device of claim 1, wherein the correction coefficient calculation part calculates a correction coefficient k by formulas (2-1) and (2-2) as follows: $k = \frac{T \times {RR}_{Au}}{{\sum }_{\overline{T}} {C}_{t}} = \frac{{RR}_{Au}}{\overline{B}}$ ${RR}_{Au} = \frac{λ \times C}{n \times ε \times Y \times {f}_{1} \times G \times \left(1-{e}^{- λ {t}_{irr}}\right) \times {e}^{- λ {t}_{c}} \times \left(1-{e}^{- λ {t}_{m}}\right)}$ where *B̅* is an average counting rate recorded by the counter; T is time for a neutron beam to irradiate the detector and the metal part, with a unit of s; RR_{Au} is a reaction rate of the metal part; C is a peak gross count of γ rays measured by the γ ray detection part within a counting time; λ is a decay constant; n is the number of targets subject to irradiation; ε is a detection efficiency of the γ ray detection part for γ rays; Y is a γ ray branching ratio; f₁ is a self-absorption correction factor of γ rays; G is a flux fluctuation correction factor; tᵢᵣᵣ is irradiation time, with a unit of s; t_{c} is cooling time, with a unit of s; and tₘ is measurement time for γ energy spectrum, with a unit of s.

4. The neutron capture therapy device of claim 3, wherein the correction system further comprises a correction part correcting the counting rate B in combination with the correction coefficient k, the corrected counting rate Bᵣ is calculated by using a formula (2-3) as follows: ${B}_{r} = B \times k$

5. The neutron capture therapy device of claim 4, wherein the neutron dose detection device further comprises a conversion unit configured to convert a counting rate recorded by the counter into a neutron flux rate or a neutron dose rate, the conversion unit calculates a corrected neutron dose rate Dᵣ by using a formula (2-4) as follows: ${D}_{r} = \frac{{B}_{r}}{σ \times {f}_{2}} \times K \times N \times CBE$ where σ is a thermal neutron reaction cross-section (cm²); f₂ is a neutron attenuation correction factor induced by an activation detector; K is a boron dose conversion factor (Gy • cm²/ppm) for flux to 1 ppm boron concentration; N is an actual boron concentration (ppm); CBE is a composite biological effect factor.

6. The neutron capture therapy device of claim 5, wherein the neutron dose detection device further comprises a neutron dose calculation unit configured to calculate the neutron flux rate or the neutron dose rate to obtain a neutron dose, the neutron dose calculation unit calculates a corrected neutron dose D_{acmr} by using a formula (2-5) as follows: ${D}_{acm r} = \sum {D}_{r}$

7. The neutron capture therapy device of claim 1, wherein the metal part is a ¹⁹⁷Au foil, and the γ ray detection part is a high-purity germanium detector.

8. The neutron capture therapy device of any one of claims 1-7, further comprising a control system configured to control the whole neutron beam irradiation process.

9. The neutron capture therapy device of claim 1, wherein the neutron beam irradiation system comprises a neutron beam generation module, and a beam adjustment module configured to adjust the neutron beam generated by the neutron beam generation module and comprising a retarder, a reflector surrounding the retarder, and a radiation shield.

10. A method for correcting a neutron capture therapy device of claim 1, the method being performed before a treatment of a patient, the method comprising: receiving neutrons of the neutron beam detected by the neutron dose detection device and outputting electrical signals;
processing **,** by the signal processing unit, the electrical signals output from the neutron dose detection device and converting the electrical signals into pulse signals; counting **,** by the counter, the pulse signals output from the signal processing unit to obtain a counting rate;
wherein the method further comprises detecting , by the γ ray detection part, γ rays emitted by the metal part after neutron activation; obtaining the reaction rate of the metal part by a measurement value of the γ rays,
calculating the correction coefficient based on the reaction rate of the metal part and the counting rate obtained by the counter, correcting, by the correction system, the counting rate obtained by the counter by the correction coefficient.

11. The method for correcting a neutron capture therapy device of claim 10, wherein the neutron capture therapy device is periodically corrected before each treatment.

12. The method for correcting a neutron capture therapy device of claim 10, further comprising: converting, by a conversion unit, the corrected counting rate of the neutron dose detection device into a neutron flux rate or a neutron dose rate.

## Patentansprüche

1. Eine Neutroneneinfangtherapievorrichtung, umfassend: eine Neutronendosis-Erfassungsvorrichtung (21) und ein Korrektursystem (3), das konfiguriert ist, die Neutronendosis-Erfassungsvorrichtung zu korrigieren, wobei die Neutronendosis-Erfassungsvorrichtung (21) einen Detektor (211), der konfiguriert ist, Neutronen des Neutronenstrahls zu empfangen und elektrische Signale auszugeben, eine Signalverarbeitungseinheit (212), die konfiguriert ist, die von dem Detektor ausgegebenen elektrischen Signale zu verarbeiten und die elektrischen Signale in Pulssignale umzuwandeln, und einen Zähler (213), der konfiguriert ist, die von der Signalverarbeitungseinheit ausgegebenen Pulssignale zu zählen, um eine Zählrate zu erhalten, umfasst; wobei das Korrektursystem (3) ein Metallteil (31), ein γ-Strahlen-Erfassungsteil (32), das konfiguriert ist, γ-Strahlen zu erfassen, die von dem durch Neutronen des Neutronenstrahls aktivierten Metallteil (31) emittiert werden, um eine Reaktionsrate des Metallteils durch einen Messwert der γ-Strahlen zu erhalten, und ein Korrekturkoeffizienten-Berechnungsteil (33), das konfiguriert ist, einen Korrekturkoeffizienten basierend auf der Reaktionsrate des Metallteils und der durch den Zähler erhaltenen Zählrate zu berechnen, umfasst, und wobei das Korrektursystem (3) konfiguriert ist, durch den Korrekturkoeffizienten die durch den Zähler erhaltene Zählrate zu korrigieren.

2. Die Neutroneneinfangtherapievorrichtung nach Anspruch 1, wobei das Korrektursystem konfiguriert ist, die Neutronendosis-Erfassungsvorrichtung periodisch zu korrigieren.

3. Die Neutroneneinfangtherapievorrichtung nach Anspruch 1, wobei das Korrekturkoeffizienten-Berechnungsteil einen Korrekturkoeffizienten k durch Formeln (2-1) und (2-2) wie folgt berechnet: $k = \frac{T \times {RR}_{Au}}{{\sum }_{T} {C}_{t}} = \frac{{RR}_{Au}}{\overline{B}}$ ${RR}_{Au} = \frac{λ \times C}{n \times ε \times Y \times {f}_{1} \times G \times \left(1-{e}^{- λ {t}_{irr}}\right) \times {e}^{- {λt}_{c}} \times \left(1-{e}^{- {λt}_{m}}\right)}$ wobei *B̅* eine durch den Zähler aufgezeichnete durchschnittliche Zählrate ist; T Zeit ist, während der ein Neutronenstrahl den Detektor und das Metallteil bestrahlt, mit einer Einheit von s; RR_{Au} eine Reaktionsrate des Metallteils ist; C eine Brutto-Spitzenzählung von γ-Strahlen ist, die durch das γ-Strahlen-Erfassungsteil innerhalb einer Zählzeit gemessen wird; λ eine Zerfallskonstante ist; n die Anzahl von einer Bestrahlung unterzogenen Targets ist; ε eine Erfassungseffizienz des γ-Strahlen-Erfassungsteils für γ-Strahlen ist; Y ein γ-Strahlen-Verzweigungsverhältnis ist; f₁ ein Selbstabsorptions-Korrekturfaktor von γ-Strahlen ist; G ein Flussfluktuations-Korrekturfaktor ist; tᵢᵣᵣ Bestrahlungszeit ist, mit einer Einheit von s; t_{c} Abkühlzeit ist, mit einer Einheit von s; und tₘ Messzeit für γ-Energiespektrum ist, mit einer Einheit von s.

4. Die Neutroneneinfangtherapievorrichtung nach Anspruch 3, wobei das Korrektursystem ferner ein Korrekturteil umfasst, das die Zählrate B in Kombination mit dem Korrekturkoeffizienten k korrigiert, wobei die korrigierte Zählrate Bᵣ berechnet wird unter Verwendung einer Formel (2-3) wie folgt: ${B}_{r} = B \times k$

5. Die Neutroneneinfangtherapievorrichtung nach Anspruch 4, wobei die Neutronendosis-Erfassungsvorrichtung ferner eine Umwandlungseinheit umfasst, die konfiguriert ist, eine durch den Zähler aufgezeichnete Zählrate in eine Neutronenflussrate oder eine Neutronendosisrate umzuwandeln, wobei die Umwandlungseinheit eine korrigierte Neutronendosisrate Dᵣ berechnet unter Verwendung einer Formel (2-4) wie folgt: ${D}_{r} = \frac{{B}_{r}}{σ \times {f}_{2}} \times K \times N \times CBE$ wobei σ ein thermischer Neutronenreaktionsquerschnitt (cm²) ist; f₂ ein durch einen Aktivierungsdetektor induzierter Neutronendämpfungs-Korrekturfaktor ist; K ein Bor-Dosisumwandlungsfaktor (Gy • cm²/ppm) für Fluss zu 1 ppm Borkonzentration ist; N eine tatsächliche Borkonzentration (ppm) ist; CBE ein zusammengesetzter biologischer Effektfaktor ist.

6. Die Neutroneneinfangtherapievorrichtung nach Anspruch 5, wobei die Neutronendosis-Erfassungsvorrichtung ferner eine Neutronendosis-Berechnungseinheit umfasst, die konfiguriert ist, die Neutronenflussrate oder die Neutronendosisrate zu berechnen, um eine Neutronendosis zu erhalten, wobei die Neutronendosis-Berechnungseinheit eine korrigierte Neutronendosis D_{acmr} berechnet unter Verwendung einer Formel (2-5) wie folgt: ${D}_{acm r} = \sum {D}_{r}$

7. Die Neutroneneinfangtherapievorrichtung nach Anspruch 1, wobei das Metallteil eine ¹⁹⁷Au-Folie ist und das γ-Strahlen-Erfassungsteil ein hochreiner Germaniumdetektor ist.

8. Die Neutroneneinfangtherapievorrichtung nach einem der Ansprüche 1-7, ferner umfassend ein Steuersystem, das konfiguriert ist, den gesamten Neutronenstrahl-Bestrahlungsprozess zu steuern.

9. Die Neutroneneinfangtherapievorrichtung nach Anspruch 1, wobei das Neutronenstrahl-Bestrahlungssystem ein Neutronenstrahlerzeugungsmodul und ein Strahlanpassungsmodul umfasst, das konfiguriert ist, den durch das Neutronenstrahlerzeugungsmodul erzeugten Neutronenstrahl anzupassen, und einen Verzögerer, einen den Verzögerer umgebenden Reflektor und eine Strahlungsabschirmung umfasst.

10. Ein Verfahren zum Korrigieren einer Neutroneneinfangtherapievorrichtung nach Anspruch 1, wobei das Verfahren vor einer Behandlung eines Patienten durchgeführt wird, wobei das Verfahren umfasst: Empfangen von Neutronen des des durch die Neutronendosis-Erfassungsvorrichtung erfassten Neutronenstrahls und Ausgeben elektrischer Signale; Verarbeiten, durch die Signalverarbeitungseinheit, der von der Neutronendosis-Erfassungsvorrichtung ausgegebenen elektrischen Signale und Umwandeln der elektrischen Signale in Pulssignale; Zählen, durch den Zähler, der von der Signalverarbeitungseinheit ausgegebenen Pulssignale, um eine Zählrate zu erhalten;
wobei das Verfahren ferner umfasst: Erfassen, durch das γ-Strahlen-Erfassungsteil, von γ-Strahlen, die von dem Metallteil nach Neutronenaktivierung emittiert werden; Erhalten der Reaktionsrate des Metallteils durch einen Messwert der γ-Strahlen, Berechnen des Korrekturkoeffizienten basierend auf der Reaktionsrate des Metallteils und der durch den Zähler erhaltenen Zählrate, Korrigieren, durch das Korrektursystem, der durch den Zähler erhaltenen Zählrate durch den Korrekturkoeffizienten.

11. Das Verfahren zum Korrigieren einer Neutroneneinfangtherapievorrichtung nach Anspruch 10, wobei die Neutroneneinfangtherapievorrichtung vor jeder Behandlung periodisch korrigiert wird.

12. Das Verfahren zum Korrigieren einer Neutroneneinfangtherapievorrichtung nach Anspruch 10, ferner umfassend: Umwandeln, durch eine Umwandlungseinheit, der korrigierten Zählrate der Neutronendosis-Erfassungsvorrichtung in eine Neutronenflussrate oder eine Neutronendosisrate.

## Revendications

1. Un dispositif de thérapie par capture de neutrons comprenant : un dispositif de détection de dose de neutrons (21) et un système de correction (3) configuré pour corriger le dispositif de détection de dose de neutrons, dans lequel le dispositif de détection de dose de neutrons (21) comprend un détecteur (211) configuré pour recevoir des neutrons du faisceau de neutrons et délivrer des signaux électriques, une unité de traitement de signal (212) configurée pour traiter les signaux électriques délivrés par le détecteur et convertir les signaux électriques en signaux d'impulsions, et un compteur (213) configuré pour compter les signaux d'impulsions délivrés par l'unité de traitement de signal afin d'obtenir un taux de comptage ; dans lequel le système de correction (3) comprend une partie métallique (31), une partie de détection de rayons γ (32) configurée pour détecter des rayons γ émis par la partie métallique (31) activée par des neutrons du faisceau de neutrons afin d'obtenir un taux de réaction de la partie métallique par une valeur de mesure des rayons γ, et une partie de calcul de coefficient de correction (33) configurée pour calculer un coefficient de correction sur la base du taux de réaction de la partie métallique et du taux de comptage obtenu par le compteur, et dans lequel le système de correction (3) est configuré pour corriger, par le coefficient de correction, le taux de comptage obtenu par le compteur.

2. Le dispositif de thérapie par capture de neutrons selon la revendication 1, dans lequel le système de correction est configuré pour corriger périodiquement le dispositif de détection de dose de neutrons.

3. Le dispositif de thérapie par capture de neutrons selon la revendication 1, dans lequel la partie de calcul de coefficient de correction calcule un coefficient de correction k par les formules (2-1) et (2-2) comme suit : $k = \frac{T \times {RR}_{Au}}{{\sum }_{T} {C}_{t}} = \frac{{RR}_{Au}}{\overline{B}}$ ${RR}_{Au} = \frac{λ \times C}{n \times ε \times Y \times {f}_{1} \times G \times \left(1-{e}^{- λ {t}_{irr}}\right) \times {e}^{- {λt}_{c}} \times \left(1-{e}^{- {λt}_{m}}\right)}$ où *B̅* est un taux de comptage moyen enregistré par le compteur ; T est un temps pendant lequel un faisceau de neutrons irradie le détecteur et la partie métallique, avec une unité de s ; RR_{Au} est un taux de réaction de la partie métallique ; C est un comptage brut de pic de rayons γ mesuré par la partie de détection de rayons γ dans un temps de comptage ; λ est une constante de décroissance ; n est le nombre de cibles soumises à irradiation ; ε est une efficacité de détection de la partie de détection de rayons γ pour des rayons γ ; Y est un rapport de branchement de rayons γ ; f₁ est un facteur de correction d'auto-absorption de rayons γ ; G est un facteur de correction de fluctuation de flux ; tᵢᵣᵣ est un temps d'irradiation, avec une unité de s ; t_{c} est un temps de refroidissement, avec une unité de s ; et tₘ est un temps de mesure pour un spectre d'énergie γ, avec une unité de s.

4. Le dispositif de thérapie par capture de neutrons selon la revendication 3, dans lequel le système de correction comprend en outre une partie de correction corrigeant le taux de comptage B en combinaison avec le coefficient de correction k, le taux de comptage corrigé Bᵣ étant calculé en utilisant une formule (2-3) comme suit : ${B}_{r} = B \times k$

5. Le dispositif de thérapie par capture de neutrons selon la revendication 4, dans lequel le dispositif de détection de dose de neutrons comprend en outre une unité de conversion configurée pour convertir un taux de comptage enregistré par le compteur en un taux de flux de neutrons ou un débit de dose de neutrons, l'unité de conversion calculant un débit de dose de neutrons corrigé Dᵣ en utilisant une formule (2-4) comme suit : ${D}_{r} = \frac{{B}_{r}}{σ \times {f}_{2}} \times K \times N \times CBE$ où σ est une section efficace de réaction de neutrons thermiques (cm²) ; f₂ est un facteur de correction d'atténuation de neutrons induite par un détecteur d'activation ; K est un facteur de conversion de dose de bore (Gy • cm²/ppm) pour un flux vers une concentration de bore de 1 ppm ; N est une concentration réelle de bore (ppm) ; CBE est un facteur d'effet biologique composite.

6. Le dispositif de thérapie par capture de neutrons selon la revendication 5, dans lequel le dispositif de détection de dose de neutrons comprend en outre une unité de calcul de dose de neutrons configurée pour calculer le taux de flux de neutrons ou le débit de dose de neutrons afin d'obtenir une dose de neutrons, l'unité de calcul de dose de neutrons calculant une dose de neutrons corrigée D_{acmr} en utilisant une formule (2-5) comme suit : ${D}_{acm r} = \sum {D}_{r}$

7. Le dispositif de thérapie par capture de neutrons selon la revendication 1, dans lequel la partie métallique est une feuille de ¹⁹⁷Au, et la partie de détection de rayons γ est un détecteur au germanium de haute pureté.

8. Le dispositif de thérapie par capture de neutrons selon l'une quelconque des revendications 1 à 7, comprenant en outre un système de commande configuré pour commander l'ensemble du processus d'irradiation par faisceau de neutrons.

9. Le dispositif de thérapie par capture de neutrons selon la revendication 1, dans lequel le système d'irradiation par faisceau de neutrons comprend un module de génération de faisceau de neutrons, et un module de réglage de faisceau configuré pour régler le faisceau de neutrons généré par le module de génération de faisceau de neutrons et comprenant un ralentisseur, un réflecteur entourant le ralentisseur, et un écran anti-rayonnement.

10. Un procédé pour corriger un dispositif de thérapie par capture de neutrons selon la revendication 1, le procédé étant exécuté avant un traitement d'un patient, le procédé comprenant : recevoir des neutrons du du faisceau de neutrons détecté par le dispositif de détection de dose de neutrons et délivrer des signaux électriques ; traiter, par l'unité de traitement de signal, les signaux électriques délivrés par le dispositif de détection de dose de neutrons et convertir les signaux électriques en signaux d'impulsions ; compter, par le compteur, les signaux d'impulsions délivrés par l'unité de traitement de signal afin d'obtenir un taux de comptage ;
dans lequel le procédé comprend en outre détecter, par la partie de détection de rayons γ, des rayons γ émis par la partie métallique après activation neutronique ; obtenir le taux de réaction de la partie métallique par une valeur de mesure des rayons γ, calculer le coefficient de correction sur la base du taux de réaction de la partie métallique et du taux de comptage obtenu par le compteur, corriger, par le système de correction, le taux de comptage obtenu par le compteur par le coefficient de correction.

11. Le procédé pour corriger un dispositif de thérapie par capture de neutrons selon la revendication 10, dans lequel le dispositif de thérapie par capture de neutrons est corrigé périodiquement avant chaque traitement.

12. Le procédé pour corriger un dispositif de thérapie par capture de neutrons selon la revendication 10, comprenant en outre : convertir, par une unité de conversion, le taux de comptage corrigé du dispositif de détection de dose de neutrons en un taux de flux de neutrons ou un débit de dose de neutrons.
